# FASCICULE DE BREVET EUROPEEN

(11) **EP 4 096 726 B1**
(45) Date de publication et mention de la délivrance du brevet: **04.03.2026**
(21) Numéro de dépôt: 21702645.9
(22) Date de dépôt: 29.01.2021
(51) Int. Cl.: A61L 2/24, F24F 3/16, G16H 40/20

(54) **SYSTÈME DE CONTRÔLE D'UN VOLUME STÉRILE**
SYSTEM ZUR STEUERUNG EINES STERILEN VOLUMENS
SYSTEM FOR CONTROLLING A STERILE VOLUME

(30) Priorité: 29.01.2020 FR 2000886
(43) Date de publication de la demande: 07.12.2022
(73) Titulaire: REMED-IA TECHNOLOGIES, 13760 Saint-Cannat (FR)
(72) Inventeur: VAISLIC, Claude, 78000 Versailles (FR); GAFSOU, Olivier, 92130 Issy-les-Moulineaux (FR)
(74) Mandataire: Cabinet Novitech
(86) Numéro de dépôt international: PCT/EP2021/052056
(87) Numéro de publication internationale: WO 2021/152059

(56) Documents cités:
- EP-A1- 2 477 168
- JP-A- 2003 076 415
- JP-A- 2003 076 415
- KR-B1- 101 789 197
- KR-B1- 101 789 197
- US-A1- 2017 004 357
- US-A1- 2017 004 357
- US-A1- 2017 004 357

## Description

La présente invention concerne un système de contrôle de l'asepsie. Plus particulièrement le système contrôle l'asepsie dans un volume stérile.

Le contrôle de l'asepsie dans le domaine chirurgical est exercé personnellement par l'intermédiaire d'une personne, pouvant être une infirmière, nommée panseuse circulante, qui exerce un contrôle visuel humain. La panseuse circulante sera par la suite mentionnée comme panseuse.

La panseuse veille à ce que les personnes réalisant une intervention chirurgicale ne viennent pas en contact avec un élément non stérile durant une intervention chirurgicale.

Si la panseuse observe une faute de l'asepsie due à un contact inapproprié de l'une des personnes participant à l'intervention chirurgicale avec un élément non stérile, elle doit annoncer la faute d'asepsie et y remédier en appliquant un protocole qui dépend de la faute. La faute d'asepsie se traduit par la rupture de l'asepsie dans le volume stérile. Cette rupture peut être sous la forme d'un contact avec un élément non stérile à l'intérieur ou à l'extérieur du volume dit stérile, ou encore l'intrusion d'un élément non stérile dans le volume stérile. Ce contact inapproprié peut concerner n'importe quel élément susceptible d'être en contact avec le patient

L'autre rôle de la panseuse circulante est d'assister les personnes réalisant l'intervention chirurgicale en leur fournissant des éléments nécessaires à la réalisation de l'intervention, tel que instruments, champs, fils agrafes, cathéters, ballons etc.

La panseuse peut être amenée à sortir de salle pour récupérer l'élément à fournir.

Lors de ce déplacement, la panseuse ne peut plus réaliser sa mission d'observation visant à déterminer s'il y a eu une faute d'asepsie.

En outre la panseuse peut ne pas avoir détecté une faute d'asepsie, exposant le patient une infection liée aux soins qui peut dans le pire des cas revêtir l'aspect d'une contamination massive de la plaie opératoire.

Les mêmes observations s'appliquent dans toutes les situations ou la surveillance d'un respect de l'asepsie rigoureuse s'impose comme la radiologie, la cardiologie interventionnelle ou l'implantation d'un cathéter en réanimation.

Il serait utile d'améliorer et de sécuriser la surveillance d'un volume stérile et la détection d'une éventuelle faute d'asepsie et ainsi assister la panseuse dans cette tâche de surveillance, par exemple lorsque la panseuse s'absente par nécessité.

La surveillance d'un volume stérile s'impose dans d'autres circonstances que lors d'une intervention chirurgicale comme dans toutes les salles blanches industrielles ou dans les opérations agroalimentaires.

Pour des raisons économiques et de sécurité, il serait utile de disposer d'un moyen de contrôle de l'asepsie ne dépendant pas intégralement des facultés humaines de surveillance d'une seule personne. US 2017/004357 A1 décrit un système de contrôle de l'asepsie d'un volume stérile.

La présente invention vise à proposer un nouveau système de contrôle de l'asepsie dans un volume stérile répondant à ce besoin.

A cet effet, un premier aspect de la présente invention a pour objet un système de contrôle de l'asepsie d'un volume stérile par exemple dans les domaines médical, industriel et agroalimentaire, selon la revendication 1.

Le système de contrôle de l'asepsie dans un volume stérile peut également comprendre une ou plusieurs des caractéristiques ci-dessous, considérées individuellement ou selon toutes les combinaisons techniquement possibles
- le système de contrôle de l'asepsie dans un volume stérile comprend au moins un capteur de contact lié à au moins un élément non stérile et configuré pour permettre la détection de tout type de contact d'un élément situé au moins en partie dans le volume stérile avec le au moins un élément non stérile, le système de détermination d'une faute d'asepsie du volume stérile étant configuré pour recevoir au moins un signal du au moins un capteur de contact et déterminer une faute d'asepsie du volume stérile sur la base des signaux reçus du au moins un capteur de contact, et/ou
- le système de contrôle comprend un dispositif d'alerte, le dispositif d'alerte étant configuré pour émettre une alerte lorsqu'une faute d'asepsie du volume stérile est déterminée, et/ou
- le dispositif d'alerte est manuellement désactivé, et/ou
- le dispositif d'alerte se réactive automatiquement, si aucune réactivation manuelle n'a eu lieu durant une période de temps prédéterminée, et/ou
- la faute d'asepsie comprend une intrusion dans le volume stérile, et/ou
- l'intrusion dans le volume stérile correspond à l'intrusion d'un élément, par exemple non stérile, dans le volume stérile, et/ou
- la faute d'asepsie comprend un contact d'un élément stérile situé dans le volume stérile avec au moins un élément non stérile, par exemple situé hors du volume stérile, et/ou
- le au moins un capteur de détection comprend une caméra, et/ou un capteur photosensible à réflexion, et/ou une douche infrarouge, et/ou un capteur de distance, et/ou
- le au moins un capteur de contact comprend un capteur chimique, et/ou une encre visible sous certaines fréquences, et/ou un capteur électrostatique, et/ou un capteur de pression, et/ou un capteur de proximité, et/ou un capteur inertiel, et/ou
- lequel le au moins un capteur de détection est localisé en dehors du volume stérile,
- le système de contrôle est utilisé dans une salle d'opération chirurgicale, et/ou
- le système de contrôle est utilisé dans une salle blanche, et/ou
- le système de contrôle comprend un dispositif de stockage d'informations configuré pour stocker au cours du temps les données du au moins un signal provenant du au moins un capteur de détection et/ou du au moins un signal provenant du au moins un capteur de contact, et/ou
- le système comprend un dispositif d'affichage, le dispositif d'affichage étant configuré pour afficher une représentation des données stockées, et/ou
- le système de contrôle comprend un système de niveaux d'autorisation configuré pour conditionner l'accès à un volume, par exemple une pièce, comprenant le volume stérile à une personne ayant un niveau d'autorisation requis, et/ou
- le système de contrôle comprend comprenant un dispositif de comptage de personnes configuré pour compter le nombre de personnes dans un volume comprenant le volume stérile, et/ou
- le système de contrôle comprend comprenant un dispositif de contrôle d'ouvertures d'une porte permettant l'accès à un volume comprenant le volume stérile,
   le dispositif de contrôle d'ouvertures de la porte est destiné à contrôler le nombre de fois que ladite, est ouverte et/ou la durée d'ouverture de ladite porte durant une période de temps donnée, et/ou
- le système de contrôle comprend un capteur de densité de particules mesurant la densité de particules en suspension dans le volume stérile et/ou un volume comprenant le volume stérile.

Un deuxième aspect de la présente invention a pour objet un procédé de contrôle de l'asepsie d'un volume stérile, par exemple dans les domaines médical, industriel et agroalimentaire, par un système de contrôle, selon la revendication 15.

Le procédé de contrôle de l'asepsie dans un volume stérile peut également comprendre une ou plusieurs des caractéristiques ci-dessous, considérées individuellement ou selon toutes les combinaisons techniquement possibles
- le système de contrôle de l'asepsie dans un volume stérile comprend au moins un capteur de contact lié à au moins un élément non stérile, et le procédé comprend :
   ∘ une étape de détection de tout type de contact d'un élément situé au moins en partie dans le volume stérile avec au moins un élément non stérile par ledit au moins un capteur de contact du système de contrôle,
   ∘ une étape de réception par le système de de détermination d'un signal relatif à un contact délivré par le au moins un capteur de contact, et
   ∘ une étape de détermination d'une faute d'asepsie du volume stérile par le système de détermination du système de contrôle sur la base des signaux reçus du au moins un capteur de contact, et/ou
- le système de contrôle comprend un dispositif d'alerte et le procédé comprend une étape d'émission d'une alerte par le dispositif d'alerte du système de contrôle lorsqu'une faute d'asepsie du volume stérile est déterminée, et/ou
- le procédé comprend une étape de désactivation manuelle du dispositif d'alerte, et/ou
- le procédé comprend une étape de réactivation automatique du dispositif d'alerte si aucune réactivation manuelle n'a eu lieu durant une période de temps prédéterminée, et/ou
- le procédé comprend une étape de détection par le au moins un capteur de détection d'une intrusion dans le volume stérile d'un élément, par exemple non stérile, et/ou
- le procédé comprend une étape de détection par le au moins un capteur de contact d'un contact d'un élément stérile situé dans le volume stérile avec au moins un élément non stérile, par exemple situé hors du volume stérile provoquant une faute d'asepsie par le au moins un capteur de contact, et/ou
- le système de contrôle comprend au moins un capteur de détection choisi parmi une caméra, et/ou un capteur photosensible à réflexion, et/ou une douche infrarouge, et/ou un capteur de distance, et/ou
- le système de contrôle comprend au moins un capteur de contact choisi parmi un capteur chimique, et/ou une encre visible sous certaines fréquences, et/ou un capteur électrostatique, et/ou un capteur de pression, et/ou un capteur de proximité, et/ou un capteur inertiel, et/ou
- le système de contrôle comprend au moins un capteur de détection localisé en dehors du volume stérile, et/ou
- le procédé de contrôle est utilisé dans une salle d'opération chirurgicale, et/ou
- le procédé de contrôle est utilisé dans une salle blanche, et/ou
- le système comprend un dispositif de stockage d'informations, et le procédé comprend une étape de stockage au cours du temps des données du au moins un signal provenant du au moins un capteur de détection et/ou du au moins un signal provenant du au moins un capteur de contact sur un dispositif de stockage d'informations, et/ou
- le système comprend un dispositif d'affichage, et le procédé comprend une étape d'affichage graphique des données stockées sur un dispositif d'affichage, et/ou
- le procédé comprend une étape d'attribution d'un niveau d'autorisation à une personne permettant l'accessibilité d'une personne à un volume, par exemple une pièce, comprenant le volume stérile ; et/ou
- le procédé comprend une étape de contrôle du nombre de personnes dans une salle d'opération, lorsque le système de contrôle détermine que le nombre de personnes comptées par le dispositif de comptage de personnes à atteint une valeur seuil, une alerte est déclenchée, et/ou
- le procédé comprend une étape de contrôle du nombres d'ouverture d'une porte de la salle d'opération, lorsque le système de contrôle détermine que le nombre d'ouverture de la porte comptées par le dispositif de comptage de d'ouvertures de porte à atteint une valeur seuil, une alerte est déclenché, et/ou
- le procédé comprend une étape de contrôle d'une densité de particules en suspension dans la salle d'opération et/ou le volume stérile, lorsque le système de contrôle détermine que la densité est supérieure à une valeur seuil, une alarme est déclenchée.

Un troisième aspect de la présente divulgation a pour objet un système de contrôle de l'asepsie d'un volume stérile par exemple dans les domaines médical, industriel et agroalimentaire, le système comprenant :
- au moins un capteur de contact lié à au moins un élément non stérile et configuré pour permettre une détection de tout type de contact d'un élément situé au moins en partie dans le volume stérile avec le au moins un élément non stérile,
un système de détermination d'une faute d'asepsie du volume stérile étant configuré pour recevoir au moins un signal du au moins un capteur de contact et déterminer une faute d'asepsie du volume stérile sur la base du au moins signal reçu du au moins un capteur de contact.

Le système de contrôle de l'asepsie dans un volume stérile peut également comprendre une ou plusieurs des caractéristiques ci-dessous, considérées individuellement ou selon toutes les combinaisons techniquement possibles :
- le système de contrôle de l'asepsie comprenant en outre une interface graphique configurée pour permettre de définir le volume stérile, en particulier les limites du volumes stérile ; et/ou
- le système de contrôle de l'asepsie dans un volume stérile comprend au moins un capteur de contact lié à au moins un élément non stérile et configuré pour permettre la détection de tout type de contact d'un élément situé au moins en partie dans le volume stérile avec le au moins un élément non stérile, le système de détermination d'une faute d'asepsie du volume stérile étant configuré pour recevoir au moins un signal du au moins un capteur de contact et déterminer une faute d'asepsie du volume stérile sur la base des signaux reçus du au moins un capteur de contact, et/ou
- le système de contrôle comprend un dispositif d'alerte, le dispositif d'alerte étant configuré pour émettre une alerte lorsqu'une faute d'asepsie du volume stérile est déterminée, et/ou
- le dispositif d'alerte est manuellement désactivé, et/ou
- le dispositif d'alerte se réactive automatiquement, si aucune réactivation manuelle n'a eu lieu durant une période de temps prédéterminée, et/ou
- la faute d'asepsie comprend une intrusion dans le volume stérile, et/ou
- l'intrusion dans le volume stérile correspond à l'intrusion d'un élément, par exemple non stérile, dans le volume stérile, et/ou
- la faute d'asepsie comprend un contact d'un élément stérile situé dans le volume stérile avec au moins un élément non stérile, par exemple situé hors du volume stérile, et/ou
- le au moins un capteur de détection comprend une caméra, et/ou un capteur photosensible à réflexion, et/ou une douche infrarouge, et/ou un capteur de distance, et/ou
- le au moins un capteur de contact comprend un capteur chimique, et/ou une encre visible sous certaines fréquences, et/ou un capteur électrostatique, et/ou un capteur de pression, et/ou un capteur de proximité, et/ou un capteur inertiel, et/ou
- lequel le au moins un capteur de détection est localisé en dehors du volume stérile,
- le système de contrôle est utilisé dans une salle d'opération chirurgicale, et/ou
- le système de contrôle est utilisé dans une salle blanche, et/ou
- le système de contrôle comprend un dispositif de stockage d'informations configuré pour stocker au cours du temps les données du au moins un signal provenant du au moins un capteur de détection et/ou du au moins un signal provenant du au moins un capteur de contact, et/ou
- le système comprend un dispositif d'affichage, le dispositif d'affichage étant configuré pour afficher une représentation des données stockées, et/ou
- le système de contrôle comprend un système de niveaux d'autorisation configuré pour conditionner l'accès à un volume, par exemple une pièce, comprenant le volume stérile à une personne ayant un niveau d'autorisation requis, et/ou
- le système de contrôle comprend comprenant un dispositif de comptage de personnes configuré pour compter le nombre de personnes dans un volume comprenant le volume stérile, et/ou
- le système de contrôle comprend comprenant un dispositif de contrôle d'ouvertures d'une porte permettant l'accès à un volume comprenant le volume stérile,
   ∘ le dispositif de contrôle d'ouvertures de la porte est destiné à contrôler le nombre de fois que ladite, est ouverte et/ou la durée d'ouverture de ladite porte durant une période de temps donnée, et/ou
- le système de contrôle comprend un capteur de densité de particules mesurant la densité de particules en suspension dans le volume stérile et/ou un volume comprenant le volume stérile.

Un quatrième aspect de la présente divulgation a pour objet un procédé de contrôle de l'asepsie d'un volume stérile, par exemple dans les domaines médical, industriel et agroalimentaire, par un système de contrôle comprenant
- au moins un capteur de contact lié à au moins un élément non stérile, et
- un système de détermination d'une faute d'asepsie du volume stérile,
le procédé comprenant :
- une étape de définition du volume stérile à l'aide d'une interface graphique, et/ou
- une étape de détection de tout type de contact d'un élément situé au moins en partie dans le volume stérile avec au moins un élément non stérile par ledit au moins un capteur de contact du système de contrôle,
- une étape de réception par le système de de détermination d'un signal relatif à un contact délivré par le au moins un capteur de contact, et
- une étape de détermination d'une faute d'asepsie du volume stérile par le système de détermination du système de contrôle sur la base d'un signal reçu du au moins un capteur de contact.

Le procédé de contrôle de l'asepsie dans un volume stérile peut également comprendre une ou plusieurs des caractéristiques ci-dessous, considérées individuellement ou selon toutes les combinaisons techniquement possibles :
- le système de contrôle comprend au moins un capteur de détection, et le procédé comprend :
   ∘ une étape de détection d'une intrusion dans le volume stérile par ledit au moins un capteur de détection du système de contrôle,
   ∘ une étape de réception par le système de détermination d'un signal relatif à une intrusion délivrée par le au moins un capteur de détection, et
   ∘ une étape de détermination d'une faute d'asepsie du volume stérile par le système de détermination du système de contrôle sur la base des signaux reçus, et/ou
- le système de contrôle comprend un dispositif d'alerte et le procédé comprend une étape d'émission d'une alerte par le dispositif d'alerte du système de contrôle lorsqu'une faute d'asepsie du volume stérile est déterminée, et/ou
- le procédé comprenant une étape de désactivation manuelle du dispositif d'alerte, et/ou
- le procédé comprenant une étape de réactivation automatique du dispositif d'alerte si aucune réactivation manuelle n'a eu lieu durant une période de temps prédéterminée, et/ou
- le procédé comprenant une étape de détection par le au moins un capteur de détection d'une intrusion dans le volume stérile d'un élément, par exemple non stérile, et/ou
- le procédé comprenant une étape de détection par le au moins un capteur de contact d'un contact d'un élément stérile situé dans le volume stérile avec au moins un élément non stérile, par exemple situé hors du volume stérile provoquant une faute d'asepsie par le au moins un capteur de contact, et/ou
- le système de contrôle comprend au moins un capteur de détection choisi parmi une caméra, et/ou un capteur photosensible à réflexion, et/ou une douche infrarouge, et/ou un capteur de distance, et/ou
- le système de contrôle comprend au moins un capteur de contact choisi parmi un capteur chimique, et/ou une encre visible sous certaines fréquences, et/ou un capteur électrostatique, et/ou un capteur de pression, et/ou un capteur de proximité, et/ou un capteur inertiel, et/ou
- le système de contrôle comprend au moins un capteur de détection localisé en dehors du volume stérile, et/ou
- le procédé de contrôle est utilisé dans une salle d'opération chirurgicale, et/ou
- le procédé de contrôle est utilisé dans une salle blanche, et/ou
- le système comprend un dispositif de stockage d'informations, et le procédé comprend une étape de stockage au cours du temps des données du au moins un signal provenant du au moins un capteur de détection et/ou du au moins un signal provenant du au moins un capteur de contact sur un dispositif de stockage d'informations, et/ou
- le système comprend un dispositif d'affichage, et le procédé comprend une étape d'affichage graphique des données stockées sur un dispositif d'affichage, et/ou
- le procédé comprend une étape d'attribution d'un niveau d'autorisation à une personne permettant l'accessibilité d'une personne à un volume, par exemple une pièce, comprenant le volume stérile ; et/ou
- le procédé comprend une étape de contrôle du nombre de personnes dans une salle d'opération, lorsque le système de contrôle détermine que le nombre de personnes comptées par le dispositif de comptage de personnes à atteint une valeur seuil, une alerte est déclenchée, et/ou
- le procédé comprend une étape de contrôle du nombres d'ouverture d'une porte de la salle d'opération, lorsque le système de contrôle détermine que le nombre d'ouverture de la porte comptées par le dispositif de comptage de d'ouvertures de porte à atteint une valeur seuil, une alerte est déclenché, et/ou
- le procédé comprend une étape de contrôle d'une densité de particules en suspension dans la salle d'opération et/ou le volume stérile, lorsque le système de contrôle détermine que la densité est supérieure à une valeur seuil, une alarme est déclenchée.

L'invention sera mieux comprise à la lumière de la description suivante dans le cadre d'une opération chirurgicale, qui n'est donnée qu'à titre indicatif et qui n'a pas pour but de limiter ladite invention, accompagnée des figures ci-dessous :
- Fig.1 est une représentation schématique d'une salle d'opération chirurgicale selon l'art antérieur,
- Fig.2 est une représentation schématique d'une salle d'opération chirurgicale selon un premier mode de réalisation,
- Fig.3 est une représentation schématique d'une salle d'opération chirurgicale selon un second mode de réalisation,
- Fig.4 est une représentation schématique partielle d'une salle d'opération chirurgicale selon un troisième mode de réalisation, et
- Fig.5 est une représentation schématique partielle d'une salle d'opération chirurgicale selon un quatrième mode de réalisation.

Sur les différentes figures, les éléments analogues sont désignés par des références identiques. En outre, les différents éléments ne sont pas nécessairement représentés à l'échelle afin de présenter une vue permettant de faciliter la compréhension de l'invention.

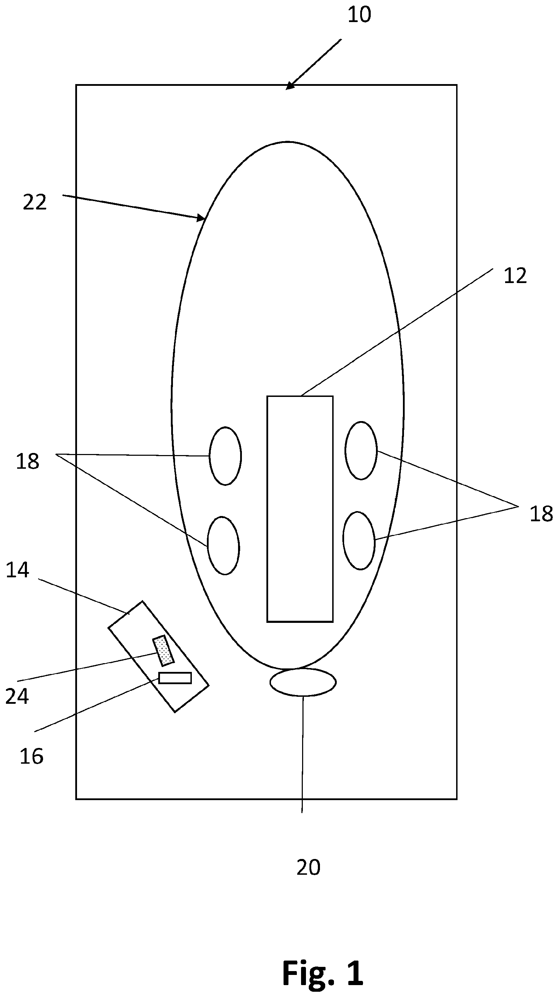

La figure 1 est une représentation schématique d'une salle d'opération chirurgicale 10 selon l'art antérieur. La salle d'opération chirurgicale 10 comprend une table d'opération 12 et une table 14 de support d'outils. Un élément 16 est disposé sur la table 14. L'élément 16 peut être plus particulièrement un instrument destiné à être utilisé par une personne 18 en charge de l'intervention chirurgicale. Une personne nommée panseuse 20 participe et assiste les personnes 18 réalisant l'intervention chirurgicale. La panseuse 20 définit un volume dit volume stérile 22. La panseuse 20 veille à ce que les personnes 18 réalisant l'intervention chirurgicale ne viennent pas en contact avec un élément non stérile 24.

L'élément non stérile 24 peut se retrouver dans le volume stérile 22 défini par la panseuse 20 comme illustré en figure 1. Mais cet élément non stérile 24, venant en contact avec au moins une des personnes 18, peut se situer en dehors du volume stérile 22.

La panseuse 20 ne peut pas assurer un contrôle permanent du volume stérile 22 si la panseuse 20 est amenée à réaliser une tâche la conduisant à quitter du regard le volume stérile 22. Il est donc nécessaire de pallier la rupture de surveillance de la panseuse 20 lorsque celle-ci réalise d'autres tâches.

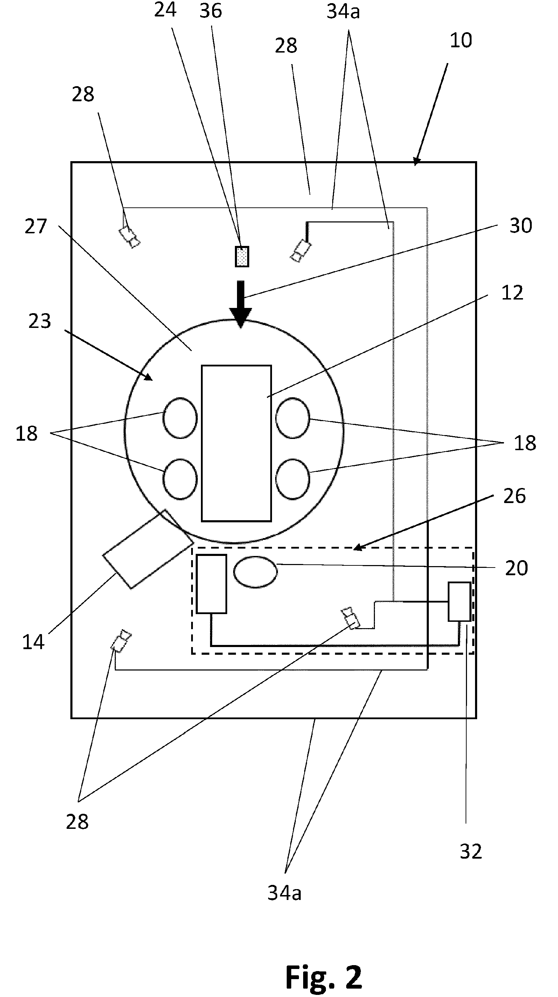

La figure 2 est une représentation schématique d'une salle d'opération chirurgicale 10 selon un premier mode de réalisation de l'invention.

De la même manière que la figure 1, la salle d'opération chirurgicale 10 illustrée en figure 2 comprend une table d'opération 12 et une table 14 de support d'outils. Un élément 16 (illustré en figure 1) peut être disposé sur la table 14. L'élément 16 peut être plus particulièrement un instrument destiné à être utilisé par une personne 18 en charge de l'intervention chirurgicale. Une panseuse 20 assiste les personnes 18 réalisant l'intervention chirurgicale. La panseuse 20 peut se situer en dehors du volume stérile 23.

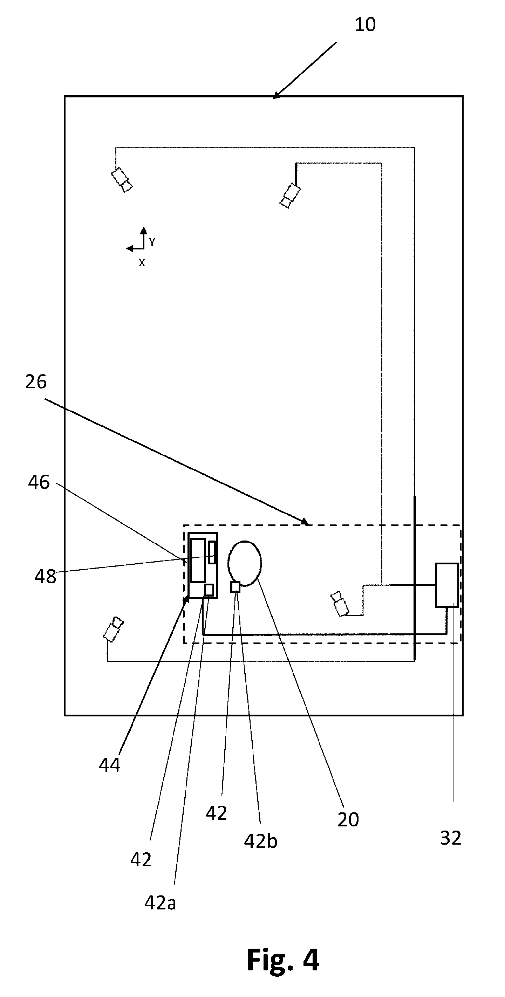

Un système de contrôle 26 de l'asepsie dans un volume stérile 23 est représenté en figure 2. Le système de contrôle 26 prédéfinit un volume stérile 23. Dans un mode de réalisation particulier, la panseuse peut prédéfinir le volume stérile 23 par le biais d'une interface graphique 46 (représenté en figure 4). Ce volume stérile 23 définit une zone sanctuarisée 27 délimitée par les contours du volume stérile 23. La zone sanctuarisée est considérée de manière distincte du volume stérile 23. De cette manière, la zone sanctuarisée 27 peut comprendre un élément non stérile 24 au sein du volume 23. La zone sanctuarisée peut en outre comprendre une personne, par exemple la panseuse 20 ou les personnes 18 réalisant l'intervention chirurgicale. De cette manière, lors de la prédéfinition du volume stérile 23, comprenant la zone sanctuarisée 27, un élément non stérile ou une personne peut être présent dans le volume stérile 23 sans déclencher une alerte par le simple fait de la présence dans le volume stérile. Les éléments disposés dans la zone sanctuarisée 27 sont considérés par le système de contrôle 26 comme étant stériles. Le système de contrôle 26 comprend au moins un capteur de détection 28. Les capteurs de détection 28 sont destinés à détecter une intrusion 30 d'un élément non stérile 24 dans le volume stérile 23.

Le système de contrôle 26 comprend en outre un système de détermination 32 d'une faute asepsie dans un volume stérile 23. Le système de détermination 32 est configuré pour recevoir au moins un signal 34a du au moins un capteur de détection 28. Lors d'une intrusion 30, dans le volume stérile 23, d'un élément extérieur 36, par exemple un élément non stérile 24 comme illustré en figure 2, le signal 34a du au moins un capteur 28 reçu par le système de détermination 32 lui permet de détecter qu'un élément extérieur 36 a été introduit dans le volume stérile 23. A partir de ce signal 34a et de la détection d'une intrusion qui en est déduite, le système de détermination 32 détermine la présence d'une faute d'asepsie du volume stérile 23.

Avantageusement un tel système de détermination 32 permet de pouvoir contrôler en permanence une éventuelle intrusion 30 dans le volume stérile 23 et ceci même si la panseuse 20 est amenée à se déplacer pour chercher un instrument ou tout élément nécessaire à la poursuite de l'intervention. Cela permet de surmonter le problème de l'absence temporaire de la panseuse dans son déplacement elle ne peut plus réaliser sa tâche de contrôle du volume stérile.

En effet, grâce à la définition préalable du volume stérile et aux traitements des données, 34a, le système 32 détermine si la zone sanctuarisée 27 a été traversée. Par exemple, si les capteurs 28 sont des caméras, le système 32, mesurera grâce à des algorithmes de traitement d'image si les frontières définissant le volume stérile 23 ont été traversées. Contrairement à un contrôle humain ce dispositif pourra vérifier l'intégrité de la zone sanctuarisée sous plusieurs points de vue en même temps.

Le système de détermination 32 peut comprendre un processus comportant une étape de traitement d'image et une étape de détection d'intrusion 30 dans le volume stérile 23. Cette étape de traitement d'image peut se faire en temps réel. Plus particulièrement, la détection de l'intrusion 30 se fait par les capteurs de détection 28.

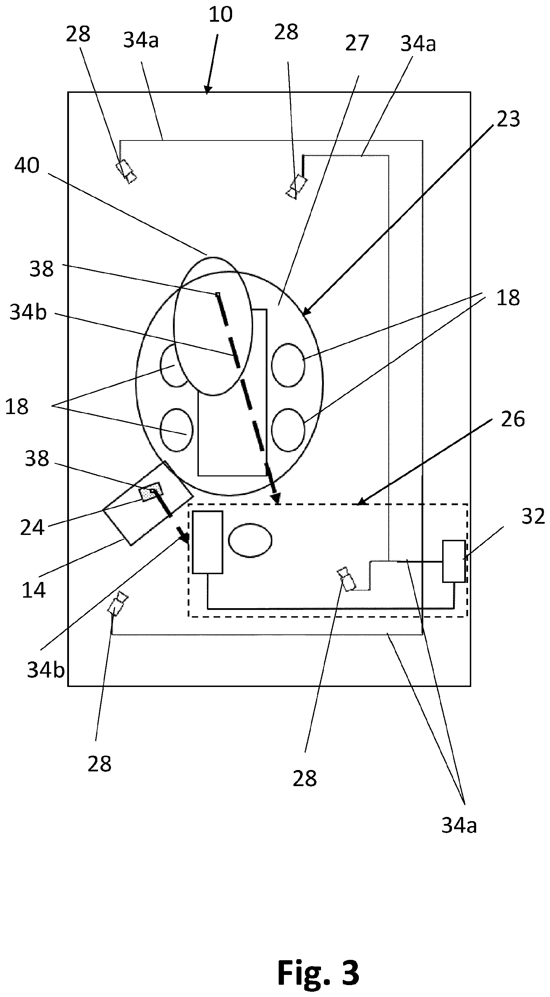

La figure 3 est une représentation schématique d'une salle d'opération chirurgicale 10 selon un second mode de réalisation comportant les caractéristiques du premier mode de réalisation illustré en figure 2.

En outre, le système de contrôle 26 comprend au moins un capteur de contact 38 lié à un élément non stérile 24. L'élément non stérile 24 peut être par exemple un instrument disposé sur la table 14 ou un scialytique 40, dont seule une poignée est stérilisée.

Une personne 18 peut être munie d'un équipement, plus particulièrement une charlotte qui pourrait venir involontairement en contact avec une partie du scialytique 40 qui n'est pas stérile.

Cet élément non stérile 24 peut se trouver dans la zone sanctuarisée 27 comme illustré en figure 3 ou au moins partiellement à l'extérieur de la zone sanctuarisée 27 et donc du volume stérile 23. L'élément non stérile 24, lié à un capteur de contact 38, peut également se situer complètement en dehors du volume stérile 23 (non représenté).

Lorsqu'un élément non stérile 24 muni d'un capteur de contact 38 entre en contact avec une personne 18, 20, un signal 34b est transmis au système de détermination 32. Le système de détermination 32 est configuré pour recevoir le signal 34b émis par le capteur de contact 38 et déterminer une faute d'asepsie sur la base des signaux 34b reçus d'au moins un capteur de contact 38.

Eventuellement, la faute d'asepsie est déterminée sur la base des signaux 34a, 34b d'au moins un capteur de contact 38 et d'au moins un capteur de détection 28.

Le au moins un capteur de contact 38 permet de détecter le contact d'une personne avec un élément non stérile 24 déjà présent dans la zone sanctuarisée 27 ou en dehors du volume stérile 23.

Ce second mode de réalisation est complémentaire du premier et permet de détecter une éventuelle faute d'asepsie indépendamment de l'intrusion 30 d'un élément dans le volume stérile 23 et en complément de la supervision de la panseuse 20.

La figure 4 est une représentation schématique partielle d'une salle d'opération chirurgicale 10.

Le système de contrôle 26 peut comprendre un dispositif d'alerte 42. Le dispositif d'alerte 42 est configuré pour émettre une alerte lorsqu'une faute d'asepsie du volume stérile est déterminée par le système de détermination 32.

Le dispositif de surveillance 44 peut comprendre le système de détermination 32.

Dans un mode de réalisation particulier, le dispositif d'alerte 42 peut également être un dispositif 42a disposé sur un dispositif de surveillance 44 situé dans la salle d'opération chirurgicale 10.

Le dispositif de surveillance 44 est un dispositif fixe, disposé préférentiellement dans la salle d'opération chirurgicale 10.

Le dispositif de surveillance 44 est compris dans le système de contrôle 26, et permet de transmettre à la panseuse 20 un signal d'alerte, à l'aide du dispositif d'alerte 42a, lorsqu'une faute d'asepsie du volume stérile 23 est déterminée.

Le signal d'alerte du dispositif d'alerte 42a peut être visuel et/ou sonore et/ou vibratoire.

Dans un mode de réalisation particulier de l'invention, le système de détermination 32 et le dispositif de surveillance 44 sont disposés dans une même enceinte.

Dans un autre mode de réalisation particulier, le dispositif d'alerte 42 peut également être un dispositif d'alerte 42b portatif.

Le dispositif d'alerte 42b est connecté par une liaison sans fil au système de détermination 32 et peut émettre un signal d'alerte visuel et/ou sonore et/ou sous la forme d'une vibration lorsqu'une faute d'asepsie du volume stérile 23 est déterminée.

Éventuellement, comme illustré en figure 4, la salle d'opération chirurgicale 10 comprend un dispositif de surveillance 44 muni d'un dispositif d'alerte 42a et la panseuse 20 dispose d'un dispositif d'alerte portatif 42b.

Le signal d'alerte peut être visuel et/ou sonore et/ou une vibration du dispositif d'alerte portatif 42b. Le dispositif portatif 42b peut être un bracelet connecté vibrant et/ou sonore, et/ou encore une tablette électronique.

Avantageusement, le dispositif d'alerte 42, 42a, 42b permet d'alerter la panseuse 20 qu'un risque de faute d'asepsie a été déterminé.

De cette manière, la panseuse 20 peut agir pour rétablir l'asepsie, par exemple elle peut indiquer à la personne 18 impliquée dans la faute d'asepsie de procéder à une procédure de stérilisation afin d'être de nouveau intégralement dans un environnement stérile.

Avantageusement, pour éviter un signal d'alerte intempestif de la part du dispositif d'alerte 42, la panseuse 20 a la possibilité de désactiver manuellement le dispositif d'alerte 42, par exemple pour permettre l'introduction volontaire d'un élément stérile dans le volume stérile 23. La panseuse 20 peut également décider manuellement de désactiver au moins un capteur de détection 28 ou de contact 38. En outre, la panseuse 20 peut également indiquer à l'aide de l'interface graphique 46 qu'une intrusion 30 d'un élément ou d'une personne dans une zone prédéfinie, à l'aide de l'interface graphique 46, du volume de stérile 23 ne constitue pas une faute d'asepsie.

Cette désactivation ponctuelle peut être faite à l'aide d'une application de téléphone, d'un programme informatique, d'un objet connecté, ou d'un actionneur mécanique ou électronique disposé sur un élément fixe du système de contrôle tel que le dispositif de surveillance 44 ou sur un élément portatif tel que le dispositif d'alerte portatif 42b, ou autre.

Le dispositif d'alerte 42 se réactive automatiquement, si aucune réactivation manuelle n'a eu lieu durant une période de temps prédéterminée.

La période de temps prédéterminée pouvant être de l'ordre de 1 minute et, plus préférentiellement de l'ordre de 30 secondes.

Avantageusement si la panseuse 20 oublie de réactiver le système d'alerte 42, le système de contrôle 26 pourra de nouveau être opérationnel. Si le système de contrôle ne s'était pas réactivé, la panseuse n'aurait pas pu bénéficier de l'assistance dans la détection d'une faute d'asepsie dans le volume stérile 23.

Dans un cas particulier de l'invention, la faute d'asepsie dans le volume stérile 23 est causée par une intrusion 30 dans le volume stérile.

Avantageusement, comme indiqué dans la description de la figure 2, une intrusion 30 dans le volume stérile 23 est détectée à l'aide d'au moins un capteur de détection 28.

Préférentiellement, le système de contrôle 26 est apte à déterminer que l'intrusion 30 dans le volume stérile 23 correspond à l'intrusion 30 d'un élément non stérile 24 dans le volume stérile 23.

Cette détermination peut être réalisée par exemple par le biais de marqueurs RFID ou de tout autre type de marqueur (codes-barres, marqueurs sensibles à une lumière spécifique, ...), qui peuvent être détectés par le capteur de détection 28, disposés sur un contenant d'un élément introduit dans le volume stérile 22.

La panseuse 20 peut également indiquer au système de contrôle 26 avant ou pendant l'intervention chirurgicale que l'élément qui va être introduit correspond à un élément stérile. De cette manière, le système de contrôle 26 est optimisé pour ne pas transmettre un signal d'alerte à la panseuse 20 lorsque l'élément introduit, est muni d'un marqueur indiquant que le contenu est stérile.

Dans un cas particulier de l'invention, la faute d'asepsie comprend un contact d'un élément stérile situé dans le volume stérile 23 avec au moins un élément non stérile 24, par exemple situé hors du volume stérile 23. Avantageusement, la présence d'un capteur de contact 38 sur les éléments non stérile 24, permet de détecter un contact de la personne 18, ou de l'un de ses équipements avec l'élément non stérile 24.

L'élément non stérile 24 muni d'un capteur de contact 38 peut se trouver au sein de la zone sanctuarisée 27 délimitée par les contours du volume stérile 23 ou bien en dehors du volume stérile 23.

Le au moins un capteur de détection 28 comprend une caméra, et/ou un capteur photosensible à réflexion, et/ou une douche infrarouge, et/ou un capteur de distance. Un agencement de plusieurs types de capteurs de détection 28 peut être considéré.

Dans un mode de réalisation, le capteur de détection 28 peut être une caméra, dont le signal correspond aux images enregistrées par la caméra ou des images anamorphosées.

La caméra peut être une caméra grand angle.

Préférablement, les capteurs de détection 28 sont au moins au nombre de trois et agencés d'une manière spécifique pour permettre une triangulation.

L'agencement des capteurs peut permettre de définir de manière tridimensionnelle un volume stérile 23 dans un premier temps et détecter en temps réel une intrusion d'un élément dans le volume stérile 23 dans un second temps.

Le volume stérile 23 peut être prédéfini préalablement par la panseuse 20 à l'aide du système de contrôle 26.

Dans un mode de réalisation particulier, le volume stérile 23 est configuré à l'aide d'une interface graphique 46 comprise dans et/ou associée au dispositif de surveillance 44.

La panseuse 20 peut redéfinir le volume stérile 23 à tout moment avant et pendant l'intervention chirurgicale. Le volume stérile 23 peut être configuré comme le volume formé par un polyèdre dont les sommets sont définis sur l'interface graphique 46.

Plusieurs autres méthodes sont également possibles pour la définition du volume stérile :
- la définition d'une forme géométrique en deux dimensions (cercle, ovale, carré rectangle, ellipse, ...) à partir d'un plan ou d'une vue de haut à l'aide d'un capteur de détection 28, de telle manière que le volume stérile 23 soit délimité en latéralité (selon un axe X illustré en figure 4) et en profondeur (selon un axe Y illustré en figure 4) par la forme géométrique en deux dimensions et en hauteur par une hauteur prédéterminée,
- le volume stérile 23 peut également être formé à partir d'une addition ou une soustraction de volumes (cylindres, sphères, cube, pavé droit,...).

Les exemples de définition du volume stérile 23 mentionnés ci-avant ne sont pas limitatives, tout autre mode de définition du volume stérile 23 peut être également considéré par la présente invention.

Dans un mode de réalisation particulier, la définition du volume stérile 23 peut être faite à partir de marqueurs type scotch QR Code disposés, par exemple par la panseuse 20, dans la salle d'opération chirurgicale 10 et détectés par les capteurs de détection 28. Le système de contrôle détecte la position des scotchs QR Code et définit le volume stérile 23. Le volume stérile 23 prédéfini est alors vérifié par la panseuse 20.

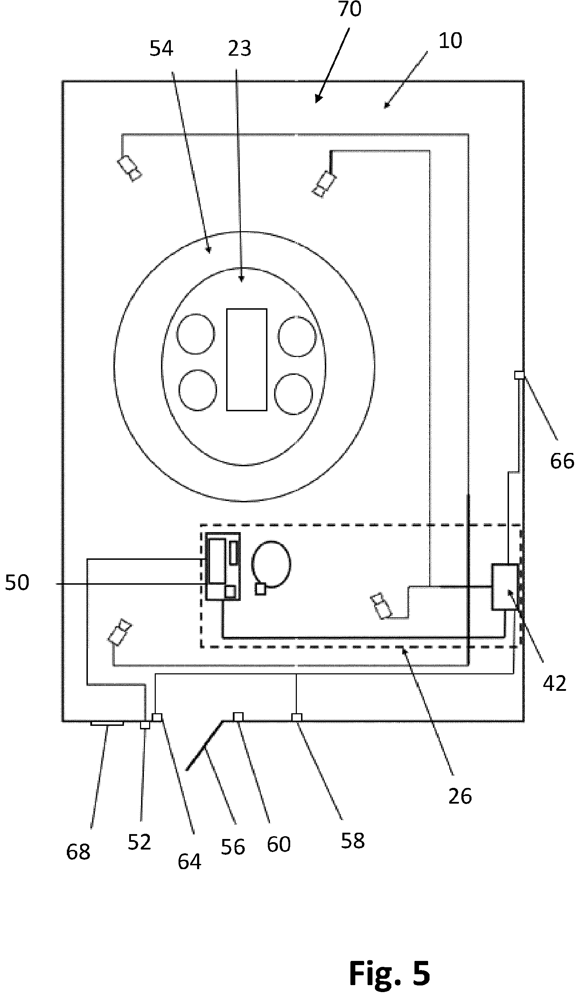

La figure 5 est une représentation schématique partielle d'une salle d'opération chirurgicale 10.

Le volume stérile 23 forme un sous volume de la salle d'opération chirurgicale 10. Le sous volume se distingue du volume de la salle d'opération chirurgicale 10 en ce qu'au moins une partie du volume de la salle d'opération chirurgicale 10 est exclue du sous volume formé par le volume stérile 23.

Ce sous volume définissant le volume stérile 23 peut être défini manuellement à l'aide du système de contrôle 26 ou à partir de marqueurs type scotch QR Code disposés, par exemple par la panseuse, dans la salle d'opération chirurgicale 10 et détectés par les capteurs de détection.

Une définition précise du volume stérile en tant que sous volume de la salle d'opération chirurgicale 10 permet de mieux déterminer toute intrusion dans le volume stérile. Le volume de stérile 23 est défini de manière à ce qu'en toute partie du volume stérile 23, un capteur de détection 28 peut détecter une intrusion.

Si pour au moins une portion du volume stérile 23, le capteur de détection 28 n'est pas apte à détecter une intrusion d'un élément non stérile 24 dans le volume stérile 23, le système de contrôle 26 pourrait ne pas détecter une faute asepsie ayant lieu dans ledit volume stérile 23.

Dans un mode de réalisation particulier, la définition du volume stérile 23 est réalisée automatiquement par le système de contrôle 26 et soumis à la validation de la panseuse 20.

Quel que soit le mode de définition du volume stérile 23, sa hauteur peut soit être définie précisément à l'aide de l'interface graphique ou par les limites spatiales de la salle d'opération chirurgicale, à savoir le sol et le plafond.

Dans un mode de mise en œuvre de l'invention, la hauteur du volume stérile 23 est définie par le sol et le plafond de la salle d'opération chirurgicale 10. Il est possible que la panseuse 20 réajuste la hauteur du volume stérile 23 de manière à ne pas inclure le scialytique 40 si ce dernier est déplacé. De cette manière, si une personne 18 manœuvre le scialytique 40 par sa poignée stérile pour l'abaisser, le scialytique 40 ne pénètre pas dans le volume stérile 23 redéfini.

Quelque soit le mode de définition du volume stérile 23, la définition peut se faire à l'aide de l'interface graphique 46 et indépendamment ou avec l'usage d'au moins une capture d'image obtenue par au moins un des capteurs de détection 28.

La détection de l'intrusion est réalisée à l'aide du système de contrôle 26 qui a permis la définition du volume stérile 23 et des capteurs de détection 28 qui par leur agencement permettent une triangulation. Le système de contrôle 26 peut mettre en œuvre un procédé de traitement d'images à partir des signaux 34a, sous forme de capture d'images. A partir de ces captures d'images en temps réel, le système de détermination 32 peut déterminer s'il y a eu une intrusion d'un élément dans le volume stérile 23 prédéfini par exemple par un algorithme de traitement d'images qui détecte le franchissement de lignes.

Dans un mode de réalisation particulier, le au moins un capteur de détection 28 comprend une caméra. Préférentiellement, plusieurs caméras pour permettre la triangulation.

Avantageusement, l'usage de caméras est élémentaire et ne demande pas une qualification importante pour la panseuse 20 ou un intervenant extérieur installant le système de contrôle 26 dans la salle d'opération chirurgicale 10. En outre, le déplacement des caméras est simple à exécuter.

Dans le cas où il y a une triangulation des caméras, un recalibrage du système peut être nécessaire à la suite du déplacement des caméras.

Le signal 34a perçu de caméra permet au système de détermination 32 de détecter une intrusion 30 dans le volume stérile 23. Préférentiellement, les caméras sont aptes à détecter des informations sur un marqueur spécifique tel qu'un autocollant et ainsi reconnaître si un élément introduit dans le volume stérile 23 est un élément stérile ou un élément non stérile 24 dépourvu du marqueur spécifique caractérisant les éléments stériles. Les caméras peuvent être facilement connectées entre elles ou au système de détermination 32 pour procéder à une triangulation.

Selon un autre mode de réalisation, au moins un capteur de détection 28 est un capteur photosensible à réflexion. Ce type de capteurs permet de définir des barrières immatérielles délimitant le volume stérile 23. Ces capteurs permettent la détection de tout type d'intrusion au travers de la barrière.

Dans le cas où une personne 18 recule suffisamment, le capteur de détection 28 détecte un franchissement en dehors du volume stérile. Le capteur 28 transmets alors un signal 34b au système de détermination 32 qui détermine alors la présence d'une faute d'asepsie. Le dispositif d'alerte 42 est configuré pour émettre une alerte lorsqu'une faute d'asepsie du volume stérile est déterminée par le dispositif de surveillance 32.

Dans le cas où une personne 18 recule suffisamment, le capteur de détection 28 détecte un franchissement en dehors du volume stérile. Le capteur 28 transmets alors un premier signal 34b, venant du capteur photosensible, au système de détermination 32. La présence d'un autre capteur de détection 28 sous la forme d'une caméra 28 permet la transmission d'un second signal 34b, venant de la caméra, au système de détermination 32. Le système de détermination 32 détermine à partir des premier et second signaux 34b combinés s'il y a réellement eu une faute d'asepsie.

Avantageusement, les capteurs photosensibles à réflexion sont très fiables.

Selon un autre mode de réalisation, au moins un capteur de détection 28 est une douche infrarouge. Un tel type de capteur est souvent utilisé pour l'ouverture de porte automatique dès qu'une personne est détectée à proximité. Avantageusement, ces capteurs sont faciles à installer et d'une grande précision.

Selon un autre mode de réalisation, au moins un capteur de détection 28 est un capteur de distance. Ces capteurs peuvent être plus particulièrement des capteurs à ultrasons qui permettent de détecter si un élément est présent proche du capteur et de déterminer s'il est proche du volume stérile 23 en déterminant sa distance vis-à-vis du volume stérile 23. Ainsi, les capteurs de distance à ultrasons peuvent être déposés sur le sol ou au plafond de manière à définir la section du volume stérile au sol. Si un élément ou une personne vient à proximité du capteur, et plus particulièrement au-dessus du capteur de distance, alors le capteur de détection 28 envoie un signal 34a au système de détermination 32 pour lui indiquer qu'une personne ou un élément s'est rapproché du capteur à une distance inférieure à une valeur seuille prédéfinie. Le système de détermination 32 détermine ainsi si une intrusion 30 dans le volume stérile 23 a eu lieu.

Selon un autre mode de réalisation, au moins un capteur de détection 28 est un capteur de distance, et plus particulièrement un capteur laser. Avantageusement les capteurs lasers sont dotés d'une précision importante.

Selon le second mode de réalisation de l'invention, le système de contrôle 26 comprend au moins un capteur de contact 38. L'au moins un capteur de contact 38 peut comprendre un capteur chimique, et/ou un capteur électrostatique, et/ou un capteur de pression, et/ou un capteur de proximité, et/ou un capteur inertiel. Les éléments non stériles 24 destinés à être munis d'un capteur de contact sont par exemple une poche de liquide, un mat ou encore un scialytique.

Selon un mode particulier du deuxième mode de réalisation, au moins un capteur de contact 38 est un capteur chimique. Les équipements d'une personne 18 peuvent être enduits d'une encre stérile invisible qui laisse une marque sur un élément non stérile 24 lorsque l'équipement enduit de la personne 18 entre en contact avec un élément non stérile 24. En combinaison avec un moyen de captations d'images, tel qu'une caméra agissante en tant que capteur de détection 28, la marque pourra être détectée par le système de contrôle 26 et un signal d'alerte informant d'une éventuelle faute asepsie sera transmis à la panseuse 20 via le dispositif d'alerte 42. La marque peut être une marque colorée ou une marque invisible détectée dans le domaine ultraviolet. Dans une alternative, ce sont les éléments non stériles 24 qui sont enduits par l'encre

Selon un autre mode de réalisation du second mode de réalisation, au moins un capteur de contact 38 est un capteur électrostatique. Ces capteurs peuvent être disposés sur les équipements des personnes 18, tel que des vêtements connectés. Tout élément stérile est défini à un même potentiel électrique et différent du potentiel électrique des éléments non stériles 24. Ainsi le contact de capteurs électrostatiques ayant un potentiel permet la transmission d'un signal 34b, via une transmission sans fil, au système de détermination 32 pour l'avertir d'un changement de potentiel. Le système de détermination 32 en déduit un contact d'une personne 18 avec un élément non stérile 24 et transmet un signal d'alerte via le dispositif d'alerte 42 pour prévenir la panseuse 20.

Selon un autre mode de réalisation du second mode de réalisation, au moins un capteur de contact 38 est un capteur de pression. Le capteur de pression est disposé sur l'élément non stérile configuré pour détecter une pression d'une personne 18 sur ledit capteur de contact 38 lors du contact avec l'élément non stérile 24. La transmission sans fil d'un signal 34b indiquant qu'une pression a été détectée par l'un des capteurs 38 permet au système de détermination 32 de déterminer qu'une personne 18 est venue en contact avec un élément non stérile 24. Le système de détermination 32 peut transmettre alors un signal d'alerte via le dispositif d'alerte 42 pour prévenir la panseuse 20.

Selon un autre mode de réalisation du second mode de réalisation, au moins un capteur de contact 38 est un capteur de proximité. Un capteur de proximité peut être positionné au niveau d'une zone à risque, et plus précisément un élément non stérile 24. Ainsi dès qu'une personne 18 se rapproche du capteur de proximité à une distance inférieure à un seuil prédéfini, un signal 34b est transmis, via une transmission sans fil, au système de détermination 32 qui déduit une faute de l'asepsie dans le volume stérile 23. Le système de détermination 32 peut transmettre alors un signal d'alerte via le dispositif d'alerte 42 pour prévenir la panseuse 20.

Selon un autre mode de réalisation du second mode de réalisation, l'au moins un capteur de contact 38 est un capteur inertiel, tel un gyroscope.

Le capteur inertiel est lié à un élément non stérile 24. De cette manière, si une personne 18 vient en contact avec l'élément non stérile 24, ce dernier subit un déplacement qui peut être mesuré par le capteur inertiel. Le capteur inertiel transmet, via une transmission sans fil, un signal 34b indiquant le déplacement de l'élément non stérile 24, au système de détermination 32. Le système de détermination 32 détermine alors qu'une personne 18 est venue en contact avec un élément non stérile 24 et peut transmettre alors un signal d'alerte via le dispositif d'alerte 42 pour prévenir la panseuse 20.

Préférentiellement, la panseuse 20 dispose un capteur de contact 38 sur chacun des éléments non stériles qui risquent de rentrer en contact avec une des personnes 18 durant l'intervention chirurgicale.

Les capteurs de contact 38 peuvent comprendre une partie adhésive pour être liée à un élément non stérile 24. Préférentiellement, les capteurs de contacts 38 sont sous forme d'autocollants.

Il est à noter que l'invention ne se limite pas aux capteurs mentionnés et couvre tout type et toute combinaison de capteurs de détection 28 et de capteurs de contacts 38. Plusieurs types de capteurs de détection 28 peuvent être utilisés dans une salle d'opération chirurgicale 10. De la même manière plusieurs types de capteurs de contact 38 peuvent être utilisés dans une salle d'opération chirurgicale 10.

L'au moins un capteur de détection 28 et l'au moins un capteur de contact 38 peuvent se situer dans ou en dehors du volume stérile 23 prédéfini.

L'au moins un capteur de détection 28 et l'au moins un capteur de contact 38 peuvent également se situer en partie à l'intérieur du volume stérile 23.

Dans un mode de réalisation particulier, le système de contrôle de l'asepsie est utilisé dans une salle d'opération chirurgicale 10.

L'objet de l'invention ne se limite pas au mode de réalisation décrit ci-avant concernant la détection d'une faute d'asepsie dans une salle d'opération chirurgicale 10.

L'invention peut aussi se rapporter au domaine industriel ou agroalimentaire

Avantageusement, le système de contrôle 26 dispose d'un dispositif de stockage d'informations 48 configuré pour stocker au cours du temps les données du au moins un signal 34a provenant du au moins un capteur de détection 28 et/ou du au moins un signal 34b provenant du au moins un capteur de contact 38.

Préférentiellement, la capture d'images obtenues par les capteurs de détection 28 est stockée en continu sur le dispositif de stockage d'informations 48 sous forme de vidéo durant l'intervention chirurgicale. De la même manière, les signaux 34b transmis par les capteurs de contact 38 sont stockés sur le dispositif de stockage d'informations 48.

Dans un mode de réalisation particulier, toutes les informations stockées sur le dispositif de stockage 48 correspondant à des signaux 34a, 34b provenant du au moins un capteur de détection 28 et/ou du au moins un capteur de contact 38 datant de plus de 6 heures sont effacées, en continu.

Préférentiellement, les informations correspondant aux signaux 34a, 34b sont stockés durant toute la durée de l'opération sans être effacées.

Dans un mode de réalisation particulier, le système de contrôle 26 comprend un dispositif d'affichage. Le dispositif d'affichage est configuré pour afficher une représentation des données stockées.

Préférentiellement, le dispositif d'affichage est formé par l'interface graphique 46. De cette manière, le système de contrôle 26 peut fournir à la panseuse 20, au moment d'une alerte, via l'interface graphique 46, une représentation graphique correspondant à chacun des signaux 34a, 34b de chacun des au moins un capteur de détection 28 et au moins un capteur de contact 38.

Préférentiellement, la représentation graphique correspondant à l'au moins un capteur de détection 28 est une capture d'image. L'image capturée par chacun des capteurs de détection 28 présents dans la salle d'opération chirurgicale 10 est affichée sur l'interface graphique 46. Concernant la représentation graphique, sur l'interface graphique 46, correspondant aux signaux 34b des capteurs de contact 38 présents dans la salle d'opération chirurgicale 10, un changement de couleur d'une chaîne de caractères peut être associé à un changement du signal 34b lié à évènement est détecté par un capteur de contact 38. Dans un autre mode de réalisation, l'affichage correspondant aux signaux 34b des capteurs de contact 38 peut être sous la forme d'un affichage spécifique, telle qu'une valeur chiffrée dès qu'un évènement est détecté par un capteur de contact 38.

Dans un mode de réalisation particulier, la panseuse 20 référence à l'aide de l'interface graphique 46 la position d'éléments non stériles 24 munis d'un capteur de contact 38. Un déplacement du au moins un capteur de contact 38 référencés se traduit alors par un clignotement ou un changement de couleur de ce dernier au niveau de l'interface graphique 46. La panseuse peut ainsi directement savoir la zone où un contact avec un objet non stérile a eu lieu. Préférentiellement, une zone autour du référencement de la position initiale de l'élément non stérile 24 muni du capteur de contact 38 est mise en surbrillance sur l'interface graphique pour indiquer la zone où le contact a eu lieu.

Dans un mode de réalisation, la représentation correspondant aux signaux 34b des capteurs de contact 38, sur l'interface graphique 46, est sous la forme d'un affichage coloré lié au dit capteur de contact 38, lorsqu'une faute d'asepsie est détectée. Préférentiellement une couleur est affectée à chaque capteur de contact 38 pour permettre à la panseuse 20 de facilement déterminer de lieu de l'éventuelle faute d'asepsie.

Dans un mode de réalisation, la panseuse 20, lors de la définition du volume stérile 23 à l'aide du système contrôle 26, indique sur la représentation comportant le volume stérile 23 la position de capteur de contact 38. Si le signal 34b émis par un capteur de détection 38 conduit à la détermination d'une faute d'asepsie par le système de détermination 32, l'interface graphique affiche une sphère, un cylindre, ou toute autre forme géométrique, autour de la position où le capteur de contact 38 est défini par la panseuse lors de la définition du volume. La forme géométrique affichée sur l'interface graphique permet d'indiquer à la panseuse une zone où la faute d'asepsie a potentiellement eu lieu.

La panseuse 20 peut à partir de ces représentations graphiques, affichées sur l'interface graphique 46, déterminer si l'alerte émise par le dispositif d'alerte 42 correspond réellement à une faute d'asepsie dans le volume stérile 23.

Dans un mode de réalisation, pour chaque alerte, la panseuse 20 peut, à partir d'une capture d'image de l'au moins un capteur de détection 28, avoir la possibilité de voir sous forme d'une vidéo de ce qui s'est passé 5 secondes avant et 5 secondes après la capture d'image d'une alerte, plus particulièrement 3 secondes avant et 3 secondes après la capture d'image. Préférentiellement, la panseuse 20 à la possibilité de stopper cette vidéo et obtenir une capture d'image à tout moment de la période de 10 secondes, plus particulièrement 6 secondes, de la vidéo. La panseuse a également la possibilité de consulter les vidéos enregistrées par chacun desdits capteurs de détection 28 à tout moment. La panseuse 20 a la possibilité de réduire la vitesse de lecture. Dans un mode de réalisation particulier la vidéo peut également être visionnée avec une lecture inversée, de manière à voir un évènement postérieur avant un évènement antérieur. La panseuse 20 peut obtenir une capture image de chacun des au moins un capteur de détection 28 lorsqu'elle met en pause la lecture de la vidéo.

Dans un mode de réalisation, pour chaque alerte, la panseuse 20 peut, à partir d'une capture d'image de l'au moins un capteur de détection 28, avoir la possibilité de voir sous forme d'une vidéo correspondant à toutes les informations enregistrées sur le dispositif de stockage d'information 48 durant l'intervention chirurgicale. La panseuse 20 a la possibilité de réduire la vitesse de lecture. Dans un mode de réalisation particulier la vidéo peut également être visionnée avec une lecture inversée, de manière à voir un évènement postérieur avant un évènement antérieur. La panseuse 20 peut obtenir une capture image de chacun des au moins un capteur de détection 28 lorsqu'elle met en pause la lecture de la vidéo.

La mise en œuvre d'un système de contrôle 26 dans une salle d'opération chirurgicale 10 comprend une première étape de mise en place du système de contrôle 26 dans la salle d'opération chirurgicale 10. Cette étape concerne particulièrement l'agencement des capteurs, et plus particulièrement des capteurs de détection 28 pour par exemple permettre une triangulation.

Une fois, le système de contrôle 26 agencé au moins pour partie dans la salle d'opération chirurgicale 10, la mise en œuvre du système de contrôle 26 comprend une deuxième étape dite étape d'initialisation. L'initialisation peut se faire en dehors du futur volume stérile 23 à définir. Alternativement, l'initialisation du système de contrôle 26 comprenant une troisième étape de définition du volume stérile 23. L'étape d'initialisation sert également à initialiser les capteurs de détection 28 et les capteurs de contact 38. Cette étape permet de vérifier que les signaux 34a, 34b des capteurs de détections 28 et de contact 38 sont bien transmis au système de contrôle 26 et vérifier que le signal d'alerte est bien transmis à la panseuse 20 en cas d'intrusion dans le volume stérile 23 ou de contact avec un élément non stérile 24.

La troisième étape consistant à la définition du volume stérile 23 peut être faite de manière manuelle via l'interface graphique 46, de manière semi-automatique en disposant des scotchs munis d'un QR Code permettant au système de contrôle de définir le volume stérile 23, ou encore la définition du volume stérile 23 peut se faire de manière complètement automatique. Que la définition du volume stérile 23 soit manuelle, semi-automatique, ou automatique, une confirmation de la panseuse 20 reste nécessaire pour s'assurer que le volume stérile 23 a bien été défini.

En outre, durant une intervention chirurgicale, la panseuse 20 peut être également amenée à intervenir sur le système de contrôle de manière à redéfinir le volume stérile 23 ou à désactiver l'alerte.

Par ailleurs, quand la panseuse 20 reçoit un signal d'alerte, la panseuse peut voir sous forme de vidéo, les évènements intervenus peu de temps avant et après correspondant à la capture d'image sélectionnée. Cette vidéo correspond au signal 34a acquis en continue par le capteur de détection 28 dont la capture d'image a été sélectionné par la panseuse 20.

En outre, dans un mode d'utilisation particulier, la panseuse 20 peut revisionner les données acquises provenant des signaux 34a, 34b des capteurs de détection 28 et de contact 38 correspondant à une alerte antérieure.

Dans un mode de réalisation préférentiel, un volume de contrôle 54 formant un second volume englobant le volume stérile 23, est défini automatiquement à partir du volume stérile 23. Le volume de contrôle 54 est défini de manière s'étendre à partir de la délimitation du volume stérile 23 d'une distance au moins supérieure ou égale à 1 mètre.

Le volume de contrôle 54 permet d'améliorer le contrôle d'une intrusion dans le volume stérile 23. En outre, la différence de volume entre le volume contrôle 54 et le volume stérile 23 définit la zone d'intervention, par exemple pour qu'un technicien intervienne sur un scialytique.

Dans un mode de réalisation particulier, le système de contrôle 26 comprend un système de niveaux d'autorisation 50 comprenant plusieurs niveaux d'autorisation. Le système d'autorisation 50 est configuré pour conditionner l'accès d'une personne à un volume donné 70.

Le volume donné 70 comprend le volume de contrôle 54 et le volume stérile 23.

Le volume donné 70 peut être une pièce, par exemple une salle d'opération 10.

Les exemples décrits par la suite faisant référence à une salle d'opération 10 pourront être applique à un volume donné 70, qui n'est pas nécessairement une salle d'opération 10.

Dans un mode de réalisation particulier, le système d'autorisation 50 est configuré pour conditionner l'accès d'une personne à différente partie de la salle d'opération 10 en fonction de son niveau d'autorisation :
- à la salle d'opération 10 en dehors d'un volume de contrôle 54 et du volume stérile 23,
- à la salle d'opération 10 et au volume de contrôle 54, en dehors du volume stérile 23, et
- à la salle d'opération 10, au volume de contrôle 54 et au volume stérile 23.

L'attribution du niveau d'autorisation à un personnel peut être défini lors de l'installation du système de contrôle, par exemple par le biais d'une base de données existante. Le niveau d'autorisation attribué à chacun des membres du personnel peut être modifié à n'importe quel moment, une fois le dispositif de contrôle 26 installé.

Dans un mode de réalisation particulier, le niveau d'autorisation peut être attribué à un personnel médical par la panseuse 20 en amont de l'opération chirurgicale.

Préférentiellement, le niveau d'autorisation d'un personnel médical peut être modifié en amont ou lors d'une opération chirurgicale, par exemple par la panseuse 20, à l'aide du système de contrôle 26, de manière à permettre audit personnel médical un accès à la salle d'opération 10 en dehors du volume de contrôle 54 et du volume stérile 23, au volume de contrôle 54 en dehors du volume stérile 23, ou encore au volume stérile 23.

Dans un mode de réalisation particulier, la panseuse 20 autorise une personne en fonction de son nom et/ou son matricule et/ou sa spécialité médicale et/ou compétence sa compétence technique.

L'attribution d'un niveau d'autorisation peut être matérialisé sous la forme d'un badge, d'un QR code, d'un code barre, un motif ayant une forme spécifique ou une couleur spécifique, ou encore une balise électronique tel qu'un capteur RFID.

L'attribution du niveau d'autorisation peut se matérialiser sur un vêtement ou sur une casaque.

Dans un autre mode de réalisation, le contrôle du niveau d'autorisation peut se réaliser par reconnaissance faciale.

Le système de niveau d'autorisation 50 peut contrôler le niveau d'autorisation d'une personne à l'aide d'au moins un moyen de contrôle 52 disposé en dehors de la salle d'opération 10 ou d'un capteur de détection 28 disposé dans la salle d'opération 10. Le moyen de contrôle 52 et/ou le capteur de détection peut être une caméra et/ou un lecteur RFID.

Le système de contrôle 26 peut déclencher une alerte, par le biais d'un dispositif d'alerte 42, si au moins un capteur de détection 28, situé dans la salle d'opération 10, détecte la présence ou l'intrusion d'une personne ayant un niveau d'autorisation non autorisé dans la salle d'opération 10, le volume de contrôle 54 ou le volume stérile 23.

Le niveau d'autorisation peut être attribué en fonction des zones d'accès qu'accorde la panseuse aux personnels soignant.

Par exemple, un personnel ayant niveau d'autorisation « niveau 1 » est autorisé à pénétrer dans la salle d'opération 10. Le personnel de niveau 1 peut être présent dans la salle d'opération 10 mais n'est pas autorisé à pénétrer dans le volume de contrôle 54 et le volume stérile 23.

Si au moins un capteur de détection 28 détecte la présence ou l'intrusion d'un personnel de niveau 1 dans le volume de contrôle 54, une alerte peut être émise par le dispositif d'alerte 42.

Un personnel ayant un niveau d'autorisation « niveau 2 » peut être autorisé à être présent dans la salle d'opération 10 et à pénétrer dans le volume de contrôle 54.

Le personnel de niveau 2 est autorisé à intervenir dans la salle d'opération 10 et dans le volume de contrôle 54, en dehors du volume stérile 23.

Si au moins un capteur de détection 28 détecte la présence ou l'intrusion d'un personnel de niveau 2 dans le volume de stérile 23, une alerte est émise par le dispositif d'alerte 42.

Par exemple, le personnel de niveau 2 est autorisé à intervenir dans une salle d'opération 10 pour régler ou réparer un dispositif technique tel qu'un scialytique 40.

Un personnel ayant un niveau d'autorisation « niveau 3 » peut être autorisé à être présent dans la salle d'opération 10, à pénétrer dans le volume de contrôle 54 et le volume stérile 23.

Le personnel de niveau 3 est typiquement le ou les médecins et/ou infirmiers présents dans la salle d'opération 10 intervenant lors de l'opération chirurgicale sur le patient.

Le système de contrôle 26 permet également l'affichage d'information en dehors de la salle d'opération chirurgicale 10. L'affichage d'information est fait à l'aide d'un moyen d'affichage 68. Le moyen d'affichage peut être un écran vidéo.

Pour garantir l'asepsie dans le volume stérile 23, il peut être intéressant de limiter l'ouverture et la fermeture d'une porte 56 de la salle d'opération 10, et ainsi limiter l'intrusion de particules en suspension, tels que des virus ou des bactéries, et contaminer le volume stérile 23.

Dans un mode de réalisation particulier le système de contrôle 26 comprend un dispositif de comptage de personnes 58 dans le volume donné 70. Le dispositif de comptage de personnes 58 est par exemple disposé près d'une porte 56 permettant l'accès au volume donné 70. Le dispositif de comptage 58 est relié au système de contrôle 26 de manière filaire ou à l'aide d'un réseau sans fil. Le dispositif de comptage 58 est relié à un capteur de détection de personne pour déterminer le nombre de personnes entrant et sortant du volume donné 70, tel qu'une salle d'opération chirurgicale 10. Le dispositif de comptage 58 est destiné à compter les personnes entrant et sortant du volume donné 70. Le système de contrôle 26 comprend une première valeur seuil liée au dispositif de comptage de personnes 58.

Dans un mode réalisation particulier, le capteur de détection de personne relié au dispositif de comptage de personnes peut être un capteur optique tel une caméra, des capteurs de pression tel que des capteurs de la technologie Technis Counting^{®}, des capteurs de distance, capteur infrarouge, capteur thermique, ou une combinaison de ces capteurs

Dans un mode de réalisation particulier, si le système de contrôle 26 détermine que le nombre de personne présent dans le volume donné 70 ou la différence entre le nombre de personnes entrées et sorties de la salle d'opération chirurgicale 10 est égale à la première valeur seuil, un premier signal d'avertissement est émis. Le premier signal d'avertissement peut être émis par le dispositif d'alerte 42. Lorsque la première valeur seuil est atteinte, le moyen d'affichage 68, disposé en dehors de la salle d'opération 10 ou au moins visible depuis l'extérieur de la salle d'opération 10, affiche un message d'information, par exemple un fond rouge et un message tel que « Entrée interdite : Danger Risques Infectieux ».

Dans un mode de réalisation particulier, le système de contrôle 26 comprend une seconde valeur seuil. La seconde valeur seuil est inférieure à la première valeur seuil. Lorsque le système de contrôle 26 détermine que la seconde valeur seuil est atteinte par le dispositif de comptage de personnes 58, un second signal d'avertissement est émis. Le second signal d'avertissement peut être émis par le dispositif d'alerte 42. Lorsque la seconde valeur seuil est atteinte, le moyen d'affichage 68, disposé en dehors de la salle d'opération 10 ou au moins visible depuis l'extérieur de la salle d'opération 10, affiche un message d'information, par exemple un fond orange et un message tel que « Une autorisation d'accès est requise pour entrer dans la salle d'opération ».

Lorsque la première valeur seuil est atteinte, la panseuse peut verrouiller manuellement la porte 56 de la salle d'opération.

Lorsque la porte 56 est verrouillée, les personnes présentes dans la salle d'opération chirurgicale 10 peuvent toujours sortir en désactivant le verrouillage de la porte 56 à l'aide d'un moyen de déverrouillage 60. Le moyen de déverrouillage 60 est situé dans la salle d'opération chirurgicale 10. Le moyen de déverrouillage est un bouton à proximité de la porte 56 ou un loquet disposé pour partie sur la porte 56.

Dans un mode de réalisation particulier, un moyen de contrôle 52 est situé à proximité de la porte 56 en dehors de la salle d'opération 10. Le moyen de contrôle permet le contrôle de l'identité et/ou de la compétence technique d'une personne désirant entre dans la salle d'opération 10. Le moyen de contrôle 52 peut une caméra ou un lecteur RFID ou une combinaison de ces moyens.

Si la première ou la seconde valeur seuil est atteinte, la panseuse 20 peut autoriser ou refuser l'accès d'une personne détectée par le moyen de contrôle 52:
- à la salle d'opération 10 en dehors d'un volume de contrôle 54 et du volume stérile 23,
- à la salle d'opération 10 et au volume de contrôle 54, en dehors du volume stérile 23, et
- à la salle d'opération 10, au volume de contrôle 54 et au volume stérile 23.

Si la panseuse décide d'autoriser la personne à accéder dans la salle d'opération 10, le moyen d'affichage 68 peut afficher un message, par exemple un fond vert et un message tel que « Vous êtes autorisée à pénétrer dans la salle d'opération. ».

La panseuse, avant d'autoriser la personne à entrer dans la salle d'opération, peut affecter à la personne entrante un niveau d'autorisation délimitant ainsi les lieux où peut intervenir la personne entrante.

Si la panseuse décide de refuser l'accès au sein de la salle d'opération 10 à la personne, le moyen d'affichage 68 affiche un message, par exemple un message sur fond rouge tel que « Vous n'êtes pas autorisée à pénétrer dans la salle d'opération. ».

Dans un mode de réalisation particulier, si la panseuse refuse l'accès à la personne détectée par le moyen de contrôle 52, la porte peut être temporairement verrouillée pour s'assurer que la personne n'entre pas dans la salle d'opération 10.

Dans un mode de réalisation, le dispositif de contrôle 26 comprend un dispositif de contrôle 64 d'ouvertures de la porte 56, le dispositif de contrôle 64 est par exemple disposé près de la porte 56. Le dispositif de contrôle 64 est relié à un capteur de détection d'ouverture de la porte 56.

Dans un mode de réalisation, lors d'une période de temps donnée, telle que la durée d'une opération chirurgicale, le dispositif de contrôle 64 est destiné à compter le nombre de fois que la porte 56 est ouverte durant une période de temps donnée, comprenant par exemple la durée de l'opération.

Dans un mode de réalisation, lors d'une période de temps donnée, telle que la durée d'une opération chirurgicale, le dispositif de contrôle 64 mesure la durée durant laquelle la porte 56 est ouverte. Préférentiellement, le système de contrôle 26 somme les durées durant laquelle la porte 56 de la salle d'opération a été ouverte.

Dans un mode réalisation particulier, capteur de détection d'ouverture de la porte 56, relié au dispositif de contrôle 64, peut être un accéléromètre placé sur la porte 56, un aimant ou un capteur de distance disposé à proximité sur une des parois du bâti de la porte 56.

Le système de contrôle 26 comprend une troisième valeur seuil, lié au dispositif de contrôle 64. Si le nombre d'ouvertures de la porte 56, la durée d'ouverture de la porte 56 ou la somme des durées d'ouvertures de la porte 56 atteint la troisième valeur seuil, un troisième signal d'avertissement est émis. Le troisième signal d'avertissement peut être émis par le dispositif d'alerte 42. Lorsque la troisième valeur seuil est atteinte, le moyen d'affichage 68, disposé en dehors de la salle d'opération 10 ou au moins visible depuis l'extérieur de la salle d'opération 10, affiche un fond rouge et un message d'information tel que « Entrée interdite : Danger Risques Infectieux ».

Dans un mode de réalisation particulier, le système de contrôle 26 comprend une quatrième valeur seuil, liée au dispositif de contrôle 64. La quatrième valeur seuil est inférieure à la troisième valeur seuil. Lorsque la quatrième valeur seuil est atteinte par le second dispositif de contrôle 64, un quatrième signal d'avertissement peut être émis par le dispositif d'alerte 42. Lorsque la quatrième valeur seuil est atteinte, le moyen d'affichage 68, disposé en dehors de la salle d'opération 10 ou au moins visible depuis l'extérieur de la salle d'opération 10, affiche un fond orange et un message d'information tel que « Une autorisation d'accès est requise pour entrer dans la salle d'opération ».

Dans un mode de réalisation le dispositif de comptage de personnes 58 et le dispositif de contrôle 64 sont formés par un seul et unique dispositif de comptage.

Dans un mode de réalisation, lorsque le moyen de contrôle 52 détecte une personne dépourvue de niveau d'autorisation, le moyen d'affichage 68 affiche un message, par exemple un message sur fond rouge tel que « Vous n'êtes pas autorisé(e) à pénétrer dans la salle d'opération. ».

Dans un mode de réalisation particulier, lorsque le moyen de contrôle 52 détecte une personne dépourvue de niveau d'autorisation, la porte 56 est temporairement verrouillée pour empêcher la personne de s'introduire dans la salle d'opération chirurgicale 10 lorsqu'une opération est en train de se dérouler.

Dans un mode réalisation, le moyen d'affichage 68 affiche systématiquement un message, par exemple un message sur fond rouge tel que « Vous n'êtes pas autorisé(e) à pénétrer dans la salle d'opération. ». Seulement lorsque le moyen de contrôle 52 détecte une personne à laquelle un niveau d'autorisation de « niveau 1 », « niveau 2 » ou « niveau 3 » a été attribué, le moyen d'affichage 68 peut afficher un message, par exemple un fond vert et un message tel que « Vous êtes autorisé(e) à pénétrer dans la salle d'opération. ».

Dans un mode réalisation, la porte 56 est systématiquement verrouillée et seulement lorsque le moyen de contrôle 52 détecte une personne de « niveau 1 », « niveau 2 » ou « niveau 3 », la porte 56 se déverrouille.

Dans un mode de réalisation particulier, le système de contrôle 26 comprend un capteur 66 mesurant une densité de particules en suspension dans la salle d'opération chirurgicale 10. Les particules en suspension peuvent être des microorganismes tels que des bactéries et/ou des virus.

Si la densité de particules en suspension dans la salle d'opération chirurgicale 10 dépasse une valeur seuil, alors un signal d'alerte est déclenché. Préférentiellement, le signal d'alerte est sonore.

Dans un mode de réalisation particulier le signal d'alerte est émis par le dispositif d'alerte 42.

Dans un mode de réalisation particulier, le système de contrôle 26 est un système holistique comprenant :
- au moins un capteur de détections 28,
- au moins un capteur de contact 38,
- le premier dispositif de comptage 58, relié à un capteur de détection, tel qu'une caméra, pour déterminer le nombre de personnes entrant et sortant dans la salle d'opération chirurgicale 10,
- le second dispositif de contrôle 64 est relié à un capteur, tel qu'un capteur de distance, pour déterminer si la porte 56 a été déplacée et par conséquent ouverte, et
- au moins un capteur 66 mesurant une densité de particules en suspension dans la salle d'opération chirurgicale 10.

Dans un mode de réalisation particulier, le système de contrôle 26 peut déclencher une alerte sur la base d'une combinaison des signaux captés par les capteurs suivants:
- capteur de détections 28,
- capteur de contact 38,
- capteur de détection, tel qu'une caméra, pour déterminer le nombre de personnes entrant et sortant dans la salle d'opération chirurgicale 10,
- un capteur, tel qu'un capteur de distance, pour déterminer si la porte 56 a été déplacée et par conséquent ouverte, et
- capteur 66 mesurant une densité de particules en suspension dans la salle d'opération chirurgicale 10.

Sur la basse de la combinaison des signaux perçus par ces capteurs, une rupture d'asepsie dans le volume stérile 23 peut être détecté et signalé.

L'invention a été décrite ci-dessus avec l'aide de modes de réalisation présentés sur des figures, sans limitation du concept inventif général.

Bien d'autres modifications et variations se suggèrent d'elles même à l'homme du métier, après réflexion sur les différents modes de réalisation illustrés dans cette demande. Ces modes de réalisation sont donnés à titre d'exemple et ne sont pas destinés à limiter la portée de l'invention, qui est déterminée exclusivement par les revendications ci-dessous.

Dans les revendications, le mot « comprenant » n'exclut pas d'autres éléments ou étapes, et l'utilisation de l'article indéfini « un » ou « une » n'exclut pas une pluralité. Le simple fait que différentes caractéristiques sont énumérées en revendications mutuellement dépendantes n'indique pas qu'une combinaison de ces caractéristiques ne puisse être avantageusement utilisée. Enfin, toute référence utilisée dans les revendications ne doit pas être interprétée comme limitation de la portée de l'invention.

## Revendications

1. Système de contrôle (26) de l'asepsie d'un volume stérile (23) dans les domaines médical, industriel et agroalimentaire, le système (26) comprenant :
- au moins un capteur de détection (28) configuré pour détecter une intrusion (30) dans le volume stérile (23), et pour délivrer un signal (34a) relatif à ladite détection,
- un système de détermination (32) d'une faute d'asepsie du volume stérile configuré pour recevoir au moins un signal (34a) du au moins un capteur de détection (28) et déterminer une faute d'asepsie du volume stérile (23) sur la base du au moins un signal (34a) reçu,
- un dispositif de surveillance (44) configuré pour permettre de transmettre un signal d'alerte lorsqu'une faute d'asepsie du volume stérile (23) est déterminée, et
- une interface graphique configurée pour permettre de définir le volume stérile (23), en particulier les limites du volumes stérile (23), ladite interface graphique étant également configurée pour permettre de redéfinir le volume stérile (23) à tout moment.

2. Système de contrôle (26) de l'asepsie d'un volume stérile (23) selon la revendication 1, comprenant au moins un capteur de contact (38) lié à au moins un élément non stérile (24) et configuré pour permettre la détection de tout type de contact d'un élément situé au moins en partie dans le volume stérile avec le au moins un élément non stérile (24),
le système de détermination (32) d'une faute d'asepsie du volume stérile (23) étant configuré pour recevoir au moins un signal (34b) du au moins un capteur de contact (38) et déterminer une faute d'asepsie du volume stérile (23) sur la base des signaux (34a, 34b) reçus du au moins un capteur de contact (38).

3. Système de contrôle (26) de l'asepsie d'un volume stérile (23) selon la revendication 1 ou 2, comprenant un dispositif d'alerte (42, 42a, 42b), le dispositif d'alerte (42, 42a, 42b) étant configuré pour émettre une alerte lorsqu'une faute d'asepsie du volume stérile (23) est déterminée.

4. Système de contrôle (26) de l'asepsie d'un volume stérile (23) selon la revendication 3, dans lequel le dispositif d'alerte (42, 42a, 42b) est configuré pour être manuellement désactivé.

5. Système de contrôle (26) de l'asepsie d'un volume stérile (23) selon la revendication 3 ou 4, dans lequel, le dispositif d'alerte (42, 42a, 42b) est configuré pour se réactiver automatiquement, si aucune réactivation manuelle n'a eu lieu durant une période de temps prédéterminée.

6. Système de contrôle (26) de l'asepsie d'un volume stérile (23) selon l'une des revendications 1 à 5, dans lequel le au moins un capteur de détection (28) comprend une caméra, et/ou un capteur photosensible à réflexion, et/ou une douche infrarouge, et/ou un capteur de distance.

7. Système de contrôle (26) de l'asepsie d'un volume stérile (23) selon l'une des revendications 2 à 6, dans lequel le au moins un capteur de contact (38) comprend un capteur chimique, et/ou un capteur électrostatique, et/ou un capteur de pression, et/ou un capteur de proximité, et/ou un capteur inertiel.

8. Système de contrôle (26) de l'asepsie d'un volume stérile (23) selon l'une des revendications 1 à 7, dans lequel le au moins un capteur de détection (28) est localisé en dehors du volume stérile (23), lorsque le volume stérile est prédéfini par le système de contrôle (26).

9. Système de contrôle (26) de l'asepsie d'un volume stérile selon l'une des revendications 1 à 8, comprenant un dispositif de stockage d'informations (48) configuré pour stocker au cours du temps les données du au moins un signal (34a) provenant du au moins un capteur de détection (28) et/ou du au moins un signal (34b) provenant du au moins un capteur de contact (38).

10. Système de contrôle (26) de l'asepsie d'un volume stérile (23) selon la revendication précédente, comprenant un dispositif d'affichage, le dispositif d'affichage étant configuré pour afficher une représentation des données stockées.

11. Système de contrôle (26) de l'asepsie d'un volume stérile (23) selon l'une des revendications précédentes, comprenant un système de niveaux d'autorisation (50) configuré pour conditionner l'accès à un volume, par exemple une pièce, comprenant le volume stérile (23) à une personne ayant un niveau d'autorisation requis.

12. Système de contrôle (26) de l'asepsie d'un volume stérile (23) selon l'une des revendications précédentes, comprenant un dispositif de comptage de personnes (58) configuré pour compter le nombre de personnes dans un volume comprenant le volume stérile (23).

13. Système de contrôle (26) de l'asepsie d'un volume stérile (23) selon l'une des revendications précédentes, comprenant un dispositif de contrôle (64) d'ouvertures d'une porte (56) permettant l'accès à un volume comprenant le volume stérile (23),
le dispositif de contrôle (64) d'ouvertures de la porte est destiné à contrôler le nombre de fois que ladite (56), est ouverte et/ou la durée d'ouverture de ladite porte durant une période de temps donnée.

14. Système de contrôle (26) de l'asepsie d'un volume stérile (23) selon l'une des revendications précédentes, comprenant un capteur de densité de particules (66) mesurant la densité de bactéries et/ou de virus en suspension dans un volume comprenant le volume stérile (23).

15. Procédé de contrôle de l'asepsie d'un volume stérile (23) par un système de contrôle (26) selon l'une quelconque des revendications précédentes, le procédé comprenant :
- une étape de détection d'une intrusion (30) dans le volume stérile (23) par ledit au moins un capteur de détection (28) du système de contrôle (26),
- une étape de réception d'un signal (34a) relatif à une intrusion (30) délivrée par le au moins un capteur de détection (28) par le système de détermination (32), et
une étape de détermination d'une faute d'asepsie du volume stérile par le système de détermination (32) du système de contrôle (26) sur la base du au moins un signal (34a) reçu, et
une étape de définition du volume stérile (23) à l'aide d'une interface graphique (46), ledit le volume stérile (23) pouvant être redéfini à tout moment par l'interface graphique.

## Patentansprüche

1. System zur Kontrolle (26) der Asepsis eines sterilen Volumens (23) im medizinischen, industriellen und Lebensmittelbereich, wobei das System (26) Folgendes beinhaltet:
- mindestens einen Detektionssensor (28), der dazu konfiguriert ist, ein Eindringen (30) in das sterile Volumen (23) zu detektieren und ein Signal (34a) bezüglich der Detektion bereitzustellen,
- ein System zur Bestimmung (32) eines Asepsisfehlers des sterilen Volumens, das dazu konfiguriert ist, mindestens ein Signal (34a) von dem mindestens einen Detektionssensor (28) zu empfangen und auf der Basis des mindestens einen empfangenen Signals (34a) einen Asepsisfehler des sterilen Volumens (23) zu bestimmen,
- eine Überwachungsvorrichtung (44), die dazu konfiguriert ist, eine Übertragung eines Warnsignals zu ermöglichen, wenn ein Asepsisfehler des sterilen Volumens (23) bestimmt wird, und
- eine grafische Benutzeroberfläche, die dazu konfiguriert ist, eine Definition des sterilen Volumens (23), insbesondere der Grenzen des sterilen Volumens (23), zu ermöglichen, wobei die grafische Benutzeroberfläche ferner dazu konfiguriert ist, jederzeit eine Neudefinition des sterilen Volumens (23) zu ermöglichen.

2. System zur Kontrolle (26) der Asepsis eines sterilen Volumens (23) nach Anspruch 1, beinhaltend mindestens einen Kontaktsensor (38), der mit mindestens einem nicht sterilen Element (24) verbunden ist und dazu konfiguriert ist, die Detektion jeglicher Art von Kontakt eines Elements, das sich mindestens teilweise in dem sterilen Volumen befindet, mit dem mindestens einen nicht sterilen Element (24) zu ermöglichen,
wobei das System zur Bestimmung (32) eines Asepsisfehlers des sterilen Volumens (23) dazu konfiguriert ist, mindestens ein Signal (34b) von dem mindestens einen Kontaktsensor (38) zu empfangen und auf der Basis der von dem mindestens einen Kontaktsensor (38) empfangenen Signale (34a, 34b) einen Asepsisfehler des sterilen Volumens (23) zu bestimmen.

3. System zur Kontrolle (26) der Asepsis eines sterilen Volumens (23) nach Anspruch 1 oder 2, beinhaltend eine Warnvorrichtung (42, 42a, 42b), wobei die Warnvorrichtung (42, 42a, 42b) dazu konfiguriert ist, eine Warnung auszugeben, wenn ein Asepsisfehler des sterilen Volumens (23) bestimmt wird.

4. System zur Kontrolle (26) der Asepsis eines sterilen Volumens (23) nach Anspruch 3, wobei die Warnvorrichtung (42, 42a, 42b) dazu konfiguriert ist, manuell deaktiviert zu werden.

5. System zur Kontrolle (26) der Asepsis eines sterilen Volumens (23) nach Anspruch 3 oder 4, wobei die Warnvorrichtung (42, 42a, 42b) dazu konfiguriert ist, sich automatisch zu reaktivieren, wenn während eines vorbestimmten Zeitraums keine manuelle Reaktivierung erfolgt ist.

6. System zur Kontrolle (26) der Asepsis eines sterilen Volumens (23) nach einem der Ansprüche 1 bis 5, wobei der mindestens eine Detektionssensor (28) eine Kamera und/oder einen Reflexionsfotosensor und/oder eine Infrarotschranke und/oder einen Abstandssensor beinhaltet.

7. System zur Kontrolle (26) der Asepsis eines sterilen Volumens (23) nach einem der Ansprüche 2 bis 6, wobei der mindestens eine Kontaktsensor (38) einen chemischen Sensor und/oder einen elektrostatischen Sensor und/oder einen Drucksensor und/oder einen Näherungssensor und/oder einen Trägheitssensor beinhaltet.

8. System zur Kontrolle (26) der Asepsis eines sterilen Volumens (23) nach einem der Ansprüche 1 bis 7, wobei sich der mindestens eine Detektionssensor (28) außerhalb des sterilen Volumens (23) befindet, wenn das sterile Volumen durch das Kontrollsystem (26) vordefiniert wird.

9. System zur Kontrolle (26) der Asepsis eines sterilen Volumens nach einem der Ansprüche 1 bis 8, beinhaltend eine Informationsspeichervorrichtung (48), die dazu konfiguriert ist, die Daten des mindestens einen Signals (34a), das von dem mindestens einen Detektionssensor (28) stammt, und/oder des mindestens einen Signals (34b), das von dem mindestens einen Kontaktsensor (38) stammt, im Laufe der Zeit zu speichern.

10. System zur Kontrolle (26) der Asepsis eines sterilen Volumens (23) nach dem vorhergehenden Anspruch, beinhaltend eine Anzeigevorrichtung, wobei die Anzeigevorrichtung dazu konfiguriert ist, eine Darstellung der gespeicherten Daten anzuzeigen.

11. System zur Kontrolle (26) der Asepsis eines sterilen Volumens (23) nach einem der vorhergehenden Ansprüche, beinhaltend ein Berechtigungsstufensystem (50), das dazu konfiguriert ist, einer Person, die eine erforderliche Berechtigungsstufe aufweist, den Zugang zu einem Volumen, beispielsweise einem Raum, der das sterile Volumen (23) beinhaltet, zu gestatten.

12. System zur Kontrolle (26) der Asepsis eines sterilen Volumens (23) nach einem der vorhergehenden Ansprüche, beinhaltend eine Personenzählvorrichtung (58), die dazu konfiguriert ist, die Anzahl von Personen in einem Volumen, welches das sterile Volumen (23) beinhaltet, zu zählen.

13. System zur Kontrolle (26) der Asepsis eines sterilen Volumens (23) nach einem der vorhergehenden Ansprüche, beinhaltend eine Vorrichtung zur Kontrolle (64) der Öffnungen einer Tür (56), die den Zugang zu einem Volumen, welches das sterile Volumen (23) beinhaltet, ermöglicht,
wobei die Vorrichtung zur Kontrolle (64) der Öffnungen der Tür dazu bestimmt ist, die Anzahl von Malen, die die Tür (56) geöffnet wird, und/oder die Öffnungsdauer der Tür während eines gegebenen Zeitraums zu kontrollieren.

14. System zur Kontrolle (26) der Asepsis eines sterilen Volumens (23) nach einem der vorhergehenden Ansprüche, beinhaltend einen Partikeldichtesensor (66), der die Dichte suspendierter Bakterien und/oder Viren in einem Volumen, welches das sterile Volumen (23) beinhaltet, misst.

15. Verfahren zur Kontrolle der Asepsis eines sterilen Volumens (23) durch ein Kontrollsystem (26) nach einem der vorhergehenden Ansprüche, wobei das Verfahren Folgendes beinhaltet:
- einen Schritt des Detektierens eines Eindringens (30) in das sterile Volumen (23) durch den mindestens einen Detektionssensor (28) des Kontrollsystems (26),
- einen Schritt des Empfangens eines Signals (34a) bezüglich eines Eindringens (30), das von dem mindestens einen Detektionssensor (28) bereitgestellt wird, durch das Bestimmungssystem (32), und
einen Schritt des Bestimmens eines Asepsisfehlers des sterilen Volumens durch das Bestimmungssystem (32) des Kontrollsystems (26) auf Basis des mindestens einen empfangenen Signals (34a) und
einen Schritt des Definierens des sterilen Volumens (23) mit Hilfe einer grafischen Benutzeroberfläche (46), wobei das sterile Volumen (23) durch die grafische Benutzeroberfläche jederzeit neu definiert werden kann.

## Claims

1. System (26) for controlling the asepsis of a sterile volume (23) in the medical, industrial and agri-food fields, the system (26) comprising:
- at least one detection sensor (28) configured to detect an intrusion (30) into the sterile volume (23), and to deliver a signal (34a) relating to said detection,
- a system (32) for determining an asepsis error in the sterile volume configured to receive at least one signal (34a) from the at least one detection sensor (28) and determine an asepsis error in the sterile volume (23) on the basis of the at least one signal (34a) received,
- a monitoring device (44) configured to make it possible to transmit an alert signal when an asepsis error in the sterile volume (23) is determined, and
- a graphical interface configured to make it possible to define the sterile volume (23), in particular the limits of the sterile volume (23), said graphical interface also being configured to make it possible to redefine the sterile volume (23) at any time.

2. System (26) for controlling the asepsis of a sterile volume (23) according to Claim 1, comprising at least one contact sensor (38) connected to at least one non-sterile element (24) and configured to allow detection of any type of contact between an element located at least partially in the sterile volume and the at least one non-sterile element (24),
the system (32) for determining an asepsis error in the sterile volume (23) being configured to receive at least one signal (34b) from the at least one contact sensor (38) and determine an asepsis error in the sterile volume (23) on the basis of the signals (34a, 34b) received from the at least one contact sensor (38).

3. System (26) for controlling the asepsis of a sterile volume (23) according to Claim 1 or 2, comprising an alert device (42, 42a, 42b), the alert device (42, 42a, 42b) being configured to emit an alert when an asepsis error in the sterile volume (23) is determined.

4. System (26) for controlling the asepsis of a sterile volume (23) according to Claim 3, wherein the alert device (42, 42a, 42b) is configured to be manually deactivated.

5. System (26) for controlling the asepsis of a sterile volume (23) according to Claim 3 or 4, wherein the alert device (42, 42a, 42b) is configured to automatically reactivate if no manual reactivation has taken place during a predetermined time period.

6. System (26) for controlling the asepsis of a sterile volume (23) according to any of Claims 1 to 5, wherein the at least one detection sensor (28) comprises a camera, and/or a reflective-type photosensitive sensor, and/or an infrared curtain, and/or a distance sensor.

7. System (26) for controlling the asepsis of a sterile volume (23) according to any of Claims 2 to 6, wherein the at least one contact sensor (38) comprises a chemical sensor, and/or an electrostatic sensor, and/or a pressure sensor, and/or a proximity sensor, and/or an inertial sensor.

8. System (26) for controlling the asepsis of a sterile volume (23) according to any of Claims 1 to 7, wherein the at least one detection sensor (28) is located outside the sterile volume (23) when the sterile volume is predefined by the control system (26).

9. System (26) for controlling the asepsis of a sterile volume according to any of Claims 1 to 8, comprising an information storage device (48) configured to store over time the data of the at least one signal (34a) originating from the at least one detection sensor (28) and/or of the at least one signal (34b) originating from the at least one contact sensor (38).

10. System (26) for controlling the asepsis of a sterile volume (23) according to the preceding claim, comprising a display device, the display device being configured to display a representation of the stored data.

11. System (26) for controlling the asepsis of a sterile volume (23) according to any of the preceding claims, comprising an authorization level system (50) configured to make access to a volume, for example a room, comprising the sterile volume (23) conditional on a person having a required authorization level.

12. System (26) for controlling the asepsis of a sterile volume (23) according to any of the preceding claims, comprising a person counting device (58) configured to count the number of persons in a volume comprising the sterile volume (23).

13. System (26) for controlling the asepsis of a sterile volume (23) according to any of the preceding claims, comprising a device (64) for controlling the opening of a door (56) allowing access to a volume comprising the sterile volume (23),
the device (64) for controlling the opening of the door is intended to control the number of times said door (56) is opened and/or the duration of opening of said door during a given time period.

14. System (26) for controlling the asepsis of a sterile volume (23) according to any of the preceding claims, comprising a particle density sensor (66) measuring the density of bacteria and/or viruses in suspension in a volume comprising the sterile volume (23).

15. Method for controlling the asepsis of a sterile volume (23) by way of a control system (26) according to any one of the preceding claims, the method comprising:
- a step of detecting an intrusion (30) into the sterile volume (23) by way of said at least one detection sensor (28) of the control system (26),
- a step of receiving a signal (34a) relating to an intrusion (30) delivered by the at least one detection sensor (28) by way of the determination system (32), and a step of determining an asepsis error in the sterile volume by way of the determination system (32) of the control system (26) on the basis of the at least one signal (34a) received, and
a step of defining the sterile volume (23) using a graphical interface (46), said sterile volume (23) being able to be redefined at any time by way of the graphical interface.
